(19) Europäisches Patentamt
European Patent Office
Office européen des brevets

(11) **EP 1 759 187 B1**

(12) **EUROPÄISCHE PATENTSCHRIFT**

(45) Veröffentlichungstag und Bekanntmachung des Hinweises auf die Patenterteilung:
**04.03.2015 Patentblatt 2015/10**

(21) Anmeldenummer: **05748773.8**

(22) Anmeldetag: **10.06.2005**

(51) Int Cl.:
*G01N 21/35* (2014.01)   *G01N 33/00* (2006.01)

(86) Internationale Anmeldenummer:
**PCT/DK2005/000381**

(87) Internationale Veröffentlichungsnummer:
**WO 2005/121751 (22.12.2005 Gazette 2005/51)**

(54) **IR-SENSOR, INSBESONDERE CO2-SENSOR**

IR-SENSOR, PARTICULARLY A CO2- SENSOR

DETECTEUR IR, EN PARTICULIER DETECTEUR DE CO2

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **14.06.2004 DE 102004028433**

(43) Veröffentlichungstag der Anmeldung:
**07.03.2007 Patentblatt 2007/10**

(73) Patentinhaber: **Danfoss Ixa A/s**
**7100 Vejle (DK)**

(72) Erfinder:
• **JENSEN, Jens, Møller**
**DK-8700 Horsens (DK)**
• **BENSLIMANE, Mohamed, Yahia**
**DK-6430 Nordborg (DK)**

(74) Vertreter: **Knoblauch, Andreas**
**Patentanwälte Dr. Knoblauch PartGmbB**
**Schlosserstrasse 23**
**60322 Frankfurt am Main (DE)**

(56) Entgegenhaltungen:
| | |
|---|---|
| WO-A-00/55602 | WO-A-97/25613 |
| GB-A- 2 176 889 | US-A- 5 005 947 |
| US-A- 5 081 998 | US-A- 5 162 658 |
| US-A- 5 306 913 | US-A- 5 612 676 |
| US-A- 5 672 874 | US-A- 5 995 008 |
| US-A1- 2003 102 434 | US-A1- 2003 147 080 |
| US-B2- 6 392 234 | |

**Beschreibung**

Die Erfindung betrifft einen IR-Sensor, insbesondere $CO_2$-Sensor, mit einer Filteranordnung, hinter der eine Detektoranordnung angeordnet ist, und einer Auswerteeinrichtung, die mit der Detektoranordnung verbunden ist, wobei die Filteranordnung einen ersten Filter und einen zweiten Filter, die als Bandpaß-Filter ausgebildet sind und jeweils ein Durchlaßband aufweisen und von denen der erste Filter ein vorbestimmtes IR-Band durchläßt und der zweite Filter nicht, und die Detektoranordnung zwei Detektoren aufweist, von denen jeder einem Filter zugeordnet ist, wobei das Durchlaßband eines Filters innerhalb des Durchlaßbandes des anderen Filters angeordnet ist und die Auswerteeinrichtung eine Differenz der Signale der Detektoren bildet.

Die Erfindung wird im folgenden anhand eines IR-Sensors für IR-absorbierende Gase beschrieben. Sie ist jedoch auch bei anderen Aufgaben anwendbar, wie später erläutert werden wird.

Ein derartiger Sensor, der als Gassensor ausgebildet ist, ist beispielsweise aus US 5 081 998 A bekannt. Dort ist eine IR-Strahlungsquelle vorgesehen, die über eine Filteranordnung insgesamt vier Detektoren beaufschlagt. Die Filteranordnung weist zwei Filter mit unterschiedlichen Durchlaßcharakteristiken auf. Ein erster Filter hat ein Durchlaßband für IR-Strahlung, die von $CO_2$ absorbiert wird. Dieser Filter wird daher auch kurz als "$CO_2$-Filter" bezeichnet. Die dahinter angeordneten Detektoren werden als $CO_2$-Detektoren bezeichnet.

Der andere Filter weist ein davon unterschiedliches Durchlaßband auf, das zur Ermittlung einer Referenzgröße dient. Die hinter diesem Referenzfilter angeordneten Detektoren werden als Referenzdetektoren bezeichnet. Zwischen der IR-Quelle und den beiden Filtern ist ein dritter Filter angeordnet, der als "natural density filter" bezeichnet wird und den ersten Filter und den zweiten Filter jeweils zur Hälfte abschattet. Dementsprechend erhält einer der beiden $CO_2$-Detektoren und einer der Referenzdetektoren nur IR-Strahlung, die sowohl durch den "natural density filter" als auch durch entweder den $CO_2$-Filter oder den Referenzfilter gelangt ist. In der Auswerteeinrichtung wird die Differenz der Ausgangssignale der beiden $CO_2$-Detektoren und die Differenz der beiden Referenzdetektoren gebildet. Die beiden Differenzen werden dann durcheinander dividiert. Einen derartigen $CO_2$-Sensor benötigt man beispielsweise bei der Bestimmung von $CO_2$ im Atem eines Patienten, um den Patienten während einer Narkose besser überwachen zu können.

Ein anderes Anwendungsfeld für Gassensoren, insbesondere $CO_2$-Sensoren, ist in US 6 369 716 B1 dargestellt. Dort dient der $CO_2$-Sensor dazu, den $CO_2$-Gehalt (Kohlendioxid-Gehalt) in einem Raum festzustellen, um mit Hilfe dieser Meßgröße das Raumklima steuern zu können.

Die $CO_2$-Konzentration in einem Raum sollte zwischen 800 ppm und 1200 ppm liegen, weil bei höheren Konzentrationen Ermüdungserscheinungen entstehen können. Die natürliche Konzentration in bebauten Gebieten liegt normalerweise bei etwa 400 ppm. Mit Hilfe eines $CO_2$-Sensors kann man ermitteln, wieviel Frischluft zugeführt werden muß, um eine gewünschte $CO_2$-Konzentration zu erzielen. Die gleichen Überlegungen gelten auch für andere Gase, bei denen ein bestimmter Gehalt nicht überschritten werden darf, beispielsweise CO (Kohlenmonoxid) oder ähnliches.

Die Erfindung wird, wie oben ausgeführt, im folgenden anhand der Messung von $CO_2$ beschrieben. Sie ist aber auch bei anderen Gasen anwendbar.

Eine Art, $CO_2$ in der Luft zu Messen, beruht auf Gasphasen-basierten Sensoren, bei denen die nondispersive, infrarote Spektroskopie (NDIR) verwendet wird. Bei dieser Art der Ermittlung des $CO_2$-Gehalts geht man davon aus, daß $CO_2$ Infrarot-Strahlung absorbiert, d.h. der Anteil der IR-Strahlung in einem bestimmten, eng umrissenen Wellenlängenbereich ist eine Größe, die man zur Bestimmung der $CO_2$-Konzentration verwenden kann.

Nachteilig bei derartigen Sensoren ist, daß sie eine relativ große Leistungsaufnahme haben. Die aus US 5 081 998 A bekannte Anordnung benötigt eine Strahlungsquelle, was sie jedenfalls bei einem längeren Gebrauch ungeeignet für eine batteriebetriebene Anwendung macht. Darüber hinaus benötigt eine derartige IR-Quelle in der Regel eine gewisse Aufheizzeit, so daß man ohne eine gewisse Vorbereitung nicht immer dann messen kann, wenn dies gewünscht ist.

Aus US2003/147080 A1 ist ein Verfahren und eine Vorrichtung zur Gasselektion bekannt. Die Vorrichtung weist eine Strahlungsquelle, einen Bereich mit einem Gas und einem Detektionsbereich auf. Im Detektionsbereich sind zwei Detektoren vorgesehen. Jedem Detektor ist jeweils ein Filter zugeordnet. In einer Ausgestaltungsform kann jeder Filter jeweils aus mehreren Elementen bzw. einem Hoch- und einem Tiefpassfilter bestehen. Die spektrale Bandbreite einer der Filter überspannt sowohl in Kurz- als auch in Langwellenrichtung einen größeren Bereich als die Bandbreite des anderen Filters. Ein Prozessor vergleicht die Ausgangssignale und der Detektoren mit einem Schwellenwert. Insbesondere kann der Prozessor dabei ein Verhältnis bilden.

Aus US 5 995 008 ist ein Feuerdetektor eine Vorrichtung, die überlappende spektrale Bänder benutzt, bekannt. Der Feuerdetektor weist einen ersten Sensor, einen zweiten Sensor auf, wobei die Sensoren in verschiedenen Spektralbereichen sensitiv sind. Eine Vergleichseinheit vergleicht die beiden Ausgangssignale der Sensoren und bildet beispielsweise ein Verhältnis oder eine Differenz aus beiden. Eine Schwellendetektionseinheit meldet Alarm, wenn ein gewisser Schwellenwert des Signalverhältnisses oder der Differenz überschritten wird. Die Spektralbereichsensivität wird durch die Auswahl eines Bandpass-Filters gewählt. Es sind zwei Spektralbereiche vorgesehen, wobei der Zweite den Ersten überlappt. Interferenzfilter unterliegen einer Transmissionsspektrumsverschiebung, die vom Winkel des einfallenden Lichts abhängt. Durch die vorgesehene Filteranordnung sei die Winkelabhängigkeit und damit die Zuverlässigkeit des Detektionssystems verringert.

Der Erfindung liegt die Aufgabe zugrunde, die Anwendung eines IR-Sensors zu vereinfachen.

Diese Aufgabe wird bei einem Gassensor der eingangs genannten Art dadurch gelöst, daß beide Filter durch aufeinanderfolgende Filterelemente gebildet sind, wobei ein Filterelement für beide Filter gleich ist und eine gemeinsame Grenzwellenlänge definiert und die Auswerteeinrichtung die Differenz der Signale auf das Signal eines Detektors normiert.

Mit dieser Ausgestaltung erreicht man, daß wesentlich mehr IR-Strahlung ausgewertet werden kann. Die IR-Strahlung wird also nicht in zwei getrennte Bereiche aufgeteilt, wobei jeder Detektor nur einen Bereich erfaßt. Vielmehr erfaßt nun der eine Detektor eine IR-Strahlung mit einem vorgegebenen Spektralbereich, der auch beispielsweise das Absorptionsspektrum des zu ermittelnden Gases, hier $CO_2$, umfaßt. Der andere Detektor erfaßt ein IR-Spektrum aus einem Teilbereich davon, das nicht mehr das Absorptionsspektrum des zu ermittelnden Gases umfaßt. Damit wird die Empfindlichkeit des Sensors erheblich gesteigert, d.h. für die Versorgung des Sensors mit IR-Strahlung werden nur noch geringere Anforderungen gestellt. Dadurch, daß man eine Differenz zwischen den Ausgangssignalen der Detektoren bildet, wird ein Störsignal, beispielsweise ein Hintergrundrauschen oder dergleichen, eliminiert. Die Normierung dieser Differenz auf das Ausgangssignal eines Detektors erlaubt es, Schwankungen in der Intensität der IR-Strahlung auszugleichen. Man kann auch mehr als zwei-Sensoren mit einer entsprechend größeren Anzahl von Filtern verwenden, wobei sich dann die einzelnen Durchlaßbereiche entsprechend überlappen. Mit einem derartigen Sensor kann man auch andere Informationen gewinnen, beispielsweise über Temperatur, eine Bewegung im Raum, Anzahl von Personen im Raum etc. Da man wesentlich mehr Strahlung erfassen kann, kann man den Leistungsverbrauch verringern, so daß man die notwendige Leistung auch über eine Batterie bereitstellen kann. Dies wiederum eröffnet größere Freiheiten bei der örtlichen Montage und Verwendung. Der Sensor kann seine Signale drahtlos übertragen.

Die beiden Filterelemente liegen in Strahlungsrichtung hintereinander, d.h. zwischen der oder den Quellen für IR-Strahlung und den Detektoren. Man kann nun die Filteranordnung so ausgestalten, daß die Startwellenlänge" durch das Filterelement definiert wird, das für beide Filter gleich ist, und die "Endwellenlänge", die das Durchlaßband begrenzt, durch die beiden anderen Filterelemente definiert wird. Dies ist eine einfache Maßnahme, um das Durchlaßband der beiden Filter mit einer vergleichsweise hohen Genauigkeit festlegen zu können.

Da beide Filter eine gemeinsame Grenzwellenlänge aufweisen, wird die Auswertung vereinfacht. Man kann dann ohne weiteres die Differenz zwischen den Ausgangssignalen der Detektoren bilden, ohne daß zusätzliche Rechenschritte erforderlich sind. Bei den Grenzwellenlängen handelt es sich um die Wellenlängen, die die Durchlaßbänder definieren, also begrenzen. Sie werden mit "Startwellenlänge" und "Endwellenlänge" bezeichnet.

Vorzugsweise ist das Durchlaßband des ersten Filters größer als das Durchlaßband des zweiten Filters. Dementsprechend umfaßt der erste Filter zusätzlich zu dem Spektralbereich, den der zweite Filter durchläßt, auch den Spektralbereich, in dem IR-Strahlung absorbiert wird.

Hierbei ist bevorzugt, daß beide Filter die gleiche Startwellenlänge aufweisen. Die "Startwellenlänge" ist die Wellenlänge, ab der die Filter eine Strahlung durchlassen. Die "gleiche" Startwellenlänge muß nicht im mathematischen Sinne identisch sein. Übliche Toleranzen, beispielsweise 5 %, sind durchaus zulässig. Derartige Toleranzen haben zwar einen Einfluß auf das Meßergebnis. Dieser Einfluß ist aber akzeptabel.

Vorteilhafterweise weist der erste Filter ein Durchlaßband auf, das um 0,3 bis 0,7 $\mu$m größer ist als das Durchlaßband des zweiten Filters. Man möchte mit dem ersten Filter im Grunde nur einen relativ engen Wellenlängen- oder Spektralbereich des IR-Spektrums abdecken, nämlich den Bereich, in dem IR-Strahlung durch $CO_2$ absorbiert wird. Der angegebene Bereich reicht dafür aus. Die Gefahr, daß Absorptionen durch andere Gase einen negativen Einfluß auf das Meßergebnis haben und dieses verfälschen, wird klein gehalten.

Hierbei ist bevorzugt, daß der erste Filter ein Durchlaßband im Bereich von 3,6 bis 4,5 $\mu$m und der zweite Filter ein Durchlaßband im Bereich von 3,6 bis 4,0 $\mu$m aufweist. Allgemein läßt sich sagen, daß der gemeinsame Spektralbereich etwa halb so groß ist wie der Spektralbereich, den der erste Filter durchläßt. In Abhängigkeit von den zu detektierenden Gasen oder anderen Größen können diese Spektralbereiche natürlich auch verschoben werden. Es hat sich aber herausgestellt, daß für $CO_2$ diese Wellenlängenbereiche günstig sind.

In einer besonders bevorzugten Ausgestaltung verwendet der Sensor die natürliche IR-Strahlung aus der Umgebung. Es ist also keine Strahlungsquelle erforderlich, die getrennt versorgt werden muß und dementsprechend eine gewisse Leistungsaufnahme hat. IR-Strahlung ist in der Regel überall vorhanden und zwar auch dann, wenn kein Sonnenlicht einstrahlt. Jeder Körper hat im Grunde eine gewisse Wärmestrahlung. Da man nun auf eine IR-Strahlungsquelle verzichten kann, erweitert sich auch der "Meßbereich", d.h. man kann größere Bereiche eines Raums auf den entsprechenden Gasgehalt hin überwachen. Dies erleichtert die Überwachung und Einstellung eines "persönlichen Raumklimas" oder der "indoor air quality". Man muß die Luft in dem Raum nicht erst zu einem Sensor führen und dort zwischen der IR-Strahlungsquelle und den Detektoren mit vorgeschalteten Filtern hindurchführen. Es reicht aus, wenn man den Sensor an einer Stelle im Raum anordnet, wo er das zu überwachende Luftvolumen sozusagen "überblicken" kann. In diesem Fall kann der Gassensor sozusagen die gemittelte Gaskonzentration auf einfache Weise erfassen. Der Sensor ermittelt also einen Durchschnittswert, was insbesondere für das persönliche Raumklima ein wesentlich besseres Meßergebnis bedeutet. Natürlich kann man mit dem Sensor auch die Technik von Sensoren verbessern, die mit Lampen oder anderen Leuchtmitteln arbeiten. Wenn man die natürliche oder Umgebungs-IR-Strahlung verwendet, kann man die Energie des Leuchtmittels reduzieren. Dies führt zu längeren Serviceintervallen und einer längeren Lebensdauer.

Vorzugsweise normiert die Auswerteeinrichtung die Differenz auf das Signal des ersten Detektors. Mit anderen Worten wird zur Normierung das Signal verwendet, das den $CO_2$-Gehalt enthält. Mit dieser Vorgehensweise erhält man eine etwas größere Dynamik.

Bevorzugterweise weisen die Filter $CaF_2$, Germanium oder Silizium auf. Das Silizium weist vorzugsweise eine Anti-Reflektionsbeschichtung auf, um den Durchgang zu verbessern.

Die Erfindung wird im folgenden anhand eines bevorzugten Ausführungsbeispiels in Verbindung mit der Zeichnung beschrieben. Hierin zeigen:

Fig. 1    eine schematische Darstellung zur Erläuterung des Funktionsprinzips der vorliegenden Erfindung,

Fig. 2    eine schematische Darstellung von zwei Durchlaßbändern zweier Filter,

Fig. 3    eine schematische Darstellung der Energiemenge, die von Detektoren erfaßt werden kann,

Fig. 4    ein Blockschaltbild zur Erläuterung des Aufbaus des Gassensors,

Fig. 5    eine schematische Darstellung des Durchlaßbereichs zweier Filter und

Fig. 6    eine schematische Darstellung einer Vorstufe eines Auswertesignals.

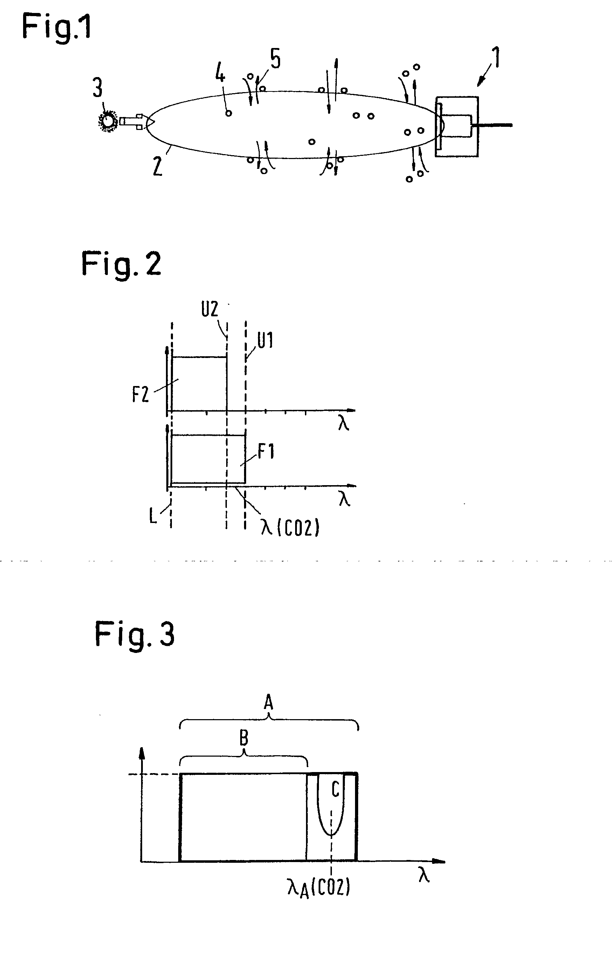

Fig. 1 zeigt in schematischer Darstellung einen Gassensor 1 zur Ermittlung des $CO_2$-Gehalts (Kohlendioxid-Gehalt) in einem Meßbereich 2. Bei dem Meßbereich kann, es sich beispielsweise um einen Raum oder den Ausschnitt eines Raumes handeln, in dem das persönliche Raumklima geregelt werden soll. Eine Sonne 3 ist als natürliche IR-Quelle dargestellt. Die Sonne 3 dient hierbei lediglich zur Erläuterung. Der Gassensor 1 arbeitet auch bei Abwesenheit von Sonnenstrahlen, weil im Prinzip praktisch jeder Körper Wärme abstrahlt und somit IR-Strahlen erzeugt.

Im Meßbereich 2 befinden sich viele $CO_2$-Moleküle, die hier als kleine Kreise dargestellt sind. Die Gasmoleküle 4 absorbieren in einem bestimmten Spektralbereich IR-Strahlen, was durch Pfeile 5 zum Ausdruck gebracht werden soll. Je größer die Konzentration von $CO_2$ ist, desto geringer ist in einem bestimmten Spektralbereich die Energie, die im Gassensor 1 erfaßt werden kann.

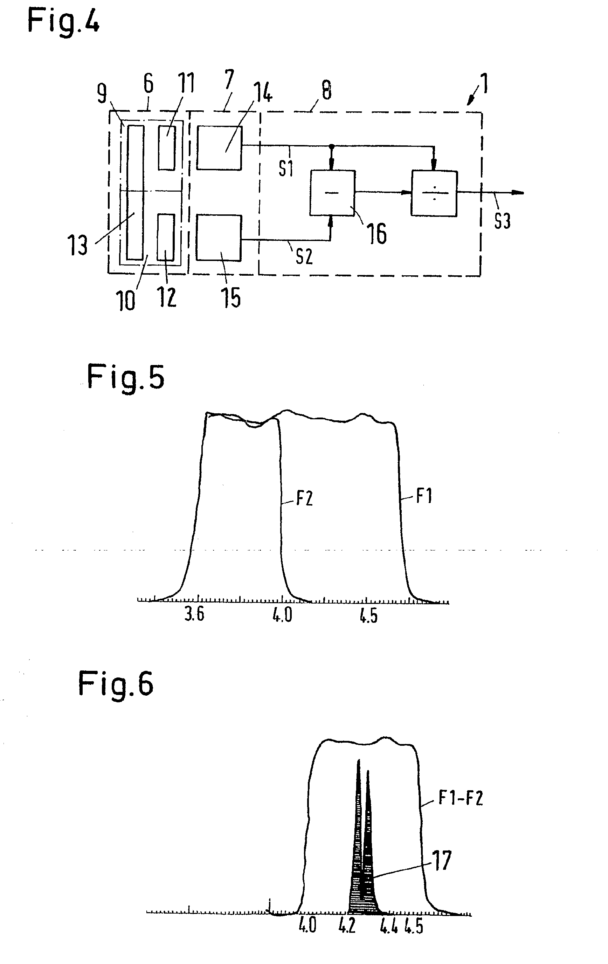

Fig. 4 zeigt nun in schematischer Darstellung ein Blockschaltbild zur Erläuterung des Aufbaus des Gassensors 1. Der Gassensor 1 weist eine Filteranordnung 6, eine Detektoranordnung 7 und eine Auswerteeinrichtung 8 auf. Weitere Einzelheiten, wie Gehäuse, Befestigungsmittel oder dergleichen, sind hier nicht dargestellt.

Die Filteranordnung weist einen ersten Filter 9 und einen zweiten Filter 10 auf. Beide Filter haben unterschiedliche Durchlaßcharakteristiken, die in Fig. 2 dargestellt sind. Der erste Filter weist ein Durchlaßband F1 auf. Der zweite Filter weist ein Durchlaßband F2 auf. Beide Durchlaßbänder F1, F2 beginnen an der gleichen unteren Grenze L, enden aber an ihrer oberen Grenze unterschiedlich und zwar der Durchlaßbereich F1 an einer oberen Grenze U1 und der Durchlaßbereich F2 an einer oberen Grenze U2. Der Abstand zwischen der oberen Grenze U1 des ersten Durchlaßbandes F1 und der oberen Grenze U2 liegt in der Größenordnung von etwa 0,3 bis etwa 0,7 $\mu$m, beispielsweise bei 0,5 $\mu$m.

In dem Bereich, in dem das Durchlaßband F1 des ersten Filters 9 größer ist als das Durchlaßband F2 des zweiten Filters 10, liegt ein Spektralbereich $\lambda$ ($CO_2$), in dem IR-Strahlung von $CO_2$ absorbiert wird. Dieser Spektralbereich liegt bei etwa 4,2 bis 4,3 $\mu$m. Dementsprechend kann man die obere Grenze U1 des ersten Durchlaßbandes F1 bei etwa 4,5 $\mu$m anordnen, die obere Grenze U2 des zweiten Durchlaßbandes F2 bei etwa 4,0 $\mu$m und die untere Grenze L, die beiden Durchlaßbändern F1, F2 gemeinsam ist, bei 3,6 $\mu$m.

Realisiert werden kann dies auf relativ einfache Weise dadurch, daß der erste Filter 9 ein erstes Filterelement 11 aufweist, das die obere Grenze U1 des Durchlaßbandes F1 definiert. Der zweite Filter 10 weist ein zweites Filterelement 12 auf, das die obere Grenze U2 des zweiten Durchlaßbandes F2 definiert. Für beide Filter 9, 10 gemeinsam ist ein drittes Filterelement 13 vorgesehen, das die untere Grenze L der beiden Durchlaßbänder F1, F2 definiert. Das dritte Filterelement 13 weist eine obere Durchlaßgrenze auf, die jenseits der oberen Grenze U1 des Durchlaßbandes F1 des ersten Filters 9 liegt. Das erste und das zweite Filterelement weisen eine untere Durchlaßgrenze auf, die unterhalb der unteren Grenze L des dritten Filterelements 13 liegt.

Dementsprechend läßt die Filteranordnung 6 im Bereich des ersten Filters 9 IR-Strahlung mit einer Energie durch, die in Fig. 3 mit A gekennzeichnet ist. Diese Energie wird um einen Anteil C vermindert, der von $CO_2$ absorbiert wird. Die Filteranordnung 6 läßt im Bereich des zweiten Filters 10 Energie passieren, die in Fig. 3 mit B markiert ist. Diese Energie ist praktisch kon-

stant, weil sie von $CO_2$ nicht beeinflußt wird.

Die unterschiedlichen Energien werden nun von der Detektoranordnung 7 erfaßt. Die Detektoranordnung 7 weist einen ersten Detektor 14 auf, der die IR-Strahlung erfaßt, die durch den ersten Filter 9 tritt, und einen zweiten Detektor 15, der die IR-Strahlung erfaßt, die durch den zweiten Filter 10 hindurchtritt. Die beiden Detektoren 14, 15 können als thermoelektrische Elemente ausgebildet sein, die auch unter der Bezeichnung "thermopiles" bekannt sind. Jeder Detektor erzeugt in Abhängigkeit von der auftretenden IR-Strahlung eine Spannung oder einen Strom, also eine elektrische Größe, die um so größer ist, je mehr IR-Strahlung auftrifft. Dementsprechend erzeugt der erste Detektor 14 ein Signal S1 und der zweite Detektor 15 ein Signal S2.

Ein Thermopile-Sensor ist beispielsweise von der PerkinElmer Optoelectronics GmbH, D-65199 Wiesbaden, Deutschland, erhältlich.

Weil in einem Thermopile-Sensor normalerweise eine Temperaturmessung erfolgt (weil das Ausgangssignal mit der Temperatur variiert), hat man schon eine Messung der Temperatur um den Sensor herum eingebaut. Da es vorstellbar ist, daß durch den Sensor auch eine Strahlungstemperatur vom Raum erhältlich ist, kann man unmittelbar aufgrund dieser beiden Messungen gleichzeitig eine Betriebstemperatur bekommen, die dann zur Steuerung der Raumtemperatur oder etwas ganz anderem angewandt werden kann.

In Verbindung mit IR ist es auch vorstellbar, daß eine Messung einer Bewegung im Raum mit dem Sensor unmittelbar möglich ist, die dann z.B. zur Steuerung einer Lüftung verwendet werden kann, die z.B. erst dann aktiviert wird, wenn eine Bewegung vorliegt, die angibt, daß sich im Raum Menschen befinden. Aufgrund verschiedener Bewegungsmessungen könnte man sich auch vorstellen, daß es möglich wäre, eine Schätzung der Anzahl von Menschen im Raum zu erhalten, wobei diese Schätzung auch für Steuerungszwecke anwendbar wäre, so daß die Raumtemperatur oder die Lüftung in Abhängigkeit der Anzahl von Personen im Raum gesteuert/geändert wird.

Beide Signale S1, S2 werden der Auswerteeinrichtung 8 zugeführt. Dabei enthalten beide Signale einen Störanteil. Man geht davon aus, daß der Störanteil für beide Detektoren 14, 15 im wesentlichen gleich ist. Dementsprechend ergibt sich dann

$$S1 = a\left(I_{CO_2} + I_n\right)$$

$$S2 = a\left(I_{ref} + I_n\right)$$

, wobei $I_{CO_2}$ die elektrische Größe, beispielsweise der Strom oder die Spannung, ist, die die Information über die IR-Strahlung enthält, während $I_{ref}$ die Referenzgröße ist, die nicht von der IR-Strahlung beeinflußt wird. Bei $I_n$ steht der Index n für "noise", also für Rauschen. Wenn man nun die Differenz zwischen S1 und S2 bildet, wofür ein Differenzbildner 16 schematisch dargestellt ist, dann erhält man eine Größe

$$S1 - S2 = a\left(I_{CO_2} - I_{ref}\right)$$

, in der der Rauschanteil verschwunden ist.

Man normiert nun diese Differenz S1 - S2 auf das Ausgangssignal S1 des ersten Detektors 14, so daß man ein Signal S3 erhält.

$$S3 = \frac{S1 - S2}{S1} = \frac{a\left(I_{CO_2} - I_{ref}\right)}{a\left(I_{CO_2} + I_n\right)}$$

Dieses Ausgangssignal S3 ist zwar wiederum durch die Störgröße $I_n$ beeinflußt. Diese Störung ist aber vernachlässigbar. Man erhält aber recht zuverlässig eine Aussage über den $CO_2$-Gehalt des Meßbereichs 2.

Fig. 5 zeigt noch einmal die unterschiedlichen Durchlaßbereiche F1, F2 der beiden Filter 9, 10, aus denen die Unterschiede zwischen den beiden Durchlaßbereichen gut zu erkennen sind.

Fig. 6 zeigt die Differenz aus den beiden Durchlaßbereichen F1, F2 und darin eingezeichnet das Absorptionsspektrum 17 für $CO_2$.

Der Gassensor ist in der Lage, $CO_2$-Konzentraionen im Bereich von etwa 300 bis etwa 1500 ppm zuverlässig zu ermitteln.

Wenn andere Gase ermittelt werden sollen, beispielsweise Stickstoff, Stickoxide, Sauerstoff oder CO, dann müssen die Durchlaßbänder entsprechend verschoben werden. In jedem Fall sollte aber gewährleistet sein, daß sich die Durchlaßbänder überlappen, um eine möglichst große Energieausbeute zu den Detektoren 14, 15 gelangen zu lassen.

Man kann vor dem Sensor auch einen Kollektor anordnen, also eine Einrichtung, die IR-Strahlung sammelt oder bündelt, beispielsweise einen Kollimator. Auch dies verbessert den Sensor.

Man kann einen derartigen Sensor auch unmittelbar zur Abgasüberwachung verwenden. Zu diesem Zweck baut man ihn in den Schornstein oder den Auspuff ein. Insbesondere bei Heizungsanlagen kann dann die Verbrennung mit Hilfe der Ausgangssignale des Sensors (oder von mehreren Sensoren) gesteuert werden.

**Patentansprüche**

**1.** IR-Sensor (1), insbesondere $CO_2$-Sensor, mit einer

Filteranordnung (6), hinter der eine Detektoranordnung (7) angeordnet ist, und einer Auswerteeinrichtung (8), die mit der Detektoranordnung verbunden ist, wobei die Filteranordnung einen ersten Filter (9) und einen zweiten Filter (10), die als Bandpaß-Filter ausgebildet sind und jeweils ein Durchlaßband aufweisen und von denen der erste Filter ein vorbestimmtes IR-Band durchläßt und der zweite Filter nicht, und die Detektoranordnung zwei Detektoren (14, 15) aufweist, von denen jeder einem Filter zugeordnet ist, wobei das Durchlaßband (F1) eines Filters innerhalb des Durchlaßbandes (F2) des anderen Filters angeordnet ist und die Auswerteeinrichtung ausgelegt ist um eine Differenz der Signale der Detektoren zubilden **dadurch gekennzeichnet, daß** beide Filter (9, 10) durch aufeinanderfolgende Filterelemente (11, 13; 12, 13) gebildet sind, wobei ein Filterelement (13) für beide Filter (9, 10) gleich ist und eine gemeinsame Grenzwellenlänge (L) definiert und die Auswerteeinrichtung (8) ausgelegt ist um die Differenz der Signale (S1, S2) auf das Signal eines Detektors (14) zu normieren.

**2.** IR-Sensor nach Anspruch 1, **dadurch gekennzeichnet, daß** das Durchlaßband (F1) des ersten Filters (9) größer ist als das Durchlaßband (F2) des zweiten Filters (10).

**3.** IR-Sensor nach Anspruch 1 oder 2, **dadurch gekennzeichnet, daß** beide Filter (9, 10) die gleiche Startwellenlänge (L) aufweisen.

**4.** IR-Sensor nach einem der Ansprüche 1 bis 3, **dadurch gekennzeichnet, daß** der erste Filter (9) ein Durchlaßband (F1) aufweist, das um 0,3 bis 0,7 $\mu$m größer ist als das Durchlaßband (F2) des zweiten Filters (10).

**5.** IR-Sensor nach Anspruch 4, **dadurch gekennzeichnet, daß** der erste Filter (9) ein Durchlaßband im Bereich von 3,6 bis 4,5 $\mu$m und der zweite Filter (10) ein Durchlaßband (F2) im Bereich von 3,6 bis 4,0 $\mu$m aufweist.

**6.** IR-Sensor nach einem der Ansprüche 1 bis 5, **dadurch gekennzeichnet, daß** er ausgelegt ist um die natürliche IR-Strahlung (3) aus der Umgebung zu verwenden.

**7.** IR-Sensor nach einem der Ansprüche 1 bis 6, **dadurch gekennzeichnet, daß** die Auswerteeinrichtung (8) ausgelegt ist um die Differenz auf das Signal (S1) des ersten Detektors (14) zu normieren.

**8.** IR-Sensor nach einem der Ansprüche 1 bis 7, **dadurch gekennzeichnet, daß** die Filter (9, 10) CaF$_2$, Germanium oder Silizium aufweisen.

**Claims**

**1.** IR sensor (1), in particular CO$_2$ sensor, having a filter arrangement (6) behind which there is arranged a detector arrangement (7), and an evaluation device (8) which is connected to the detector arrangement, wherein the filter arrangement has a first filter (9) and a second filter (10), which are formed as band-pass filters and each have a pass band, and of which the first filter allows a predetermined IR band through and the second filter does not, and the detector arrangement has two detectors (14, 15), each of which is assigned to a filter, wherein the pass band (F1) of one filter is arranged within the pass band (F2) of the other filter and the evaluation device is able to form a difference between the signals from the detectors, **characterized in that** both filters (9, 10) are formed by successive filter elements (11, 13; 12, 13), wherein one filter element (13) is the same for both filters (9, 10) and defines a common limiting wavelength (L), and the evaluation device (8) is able to normalize the difference between the signals (S1, S2) to the signal from one detector (14).

**2.** IR sensor according to Claim 1, **characterized in that** the pass band (F1) of the first filter (9) is larger than the pass band (F2) of the second filter (10).

**3.** IR sensor according to Claim 1 or 2, **characterized in that** both filters (9, 10) have the same starting wavelength (L).

**4.** IR sensor according to one of Claims 1 to 3, **characterized in that** the first filter (9) has a pass band (F1) which is 0.3 to 0.7 $\mu$m larger than the pass band (F2) of the second filter (10).

**5.** IR sensor according to Claim 4, **characterized in that** the first filter (9) has a pass band in the range from 3.6 to 4.5 $\mu$m, and the second filter (10) has a pass band (F2) in the range from 3.6 to 4.0 $\mu$m.

**6.** IR sensor according to one of claims 1 to 5, **characterized in that** it is able to use the natural IR radiation from the environment.

**7.** IR sensor according to one of Claims 1 to 6, **characterized in that** the evaluation device (8) is able to normalize the difference to the signal (S1) from the first detector (14).

**8.** IR sensor according to one of Claims 1 to 7, **characterized in that** the filters (9, 10) have CaF$_2$, germanium or silicon

**Revendications**

1. Détecteur IR (1), en particulier détecteur de $CO_2$, avec un agencement de filtres (6) derrière lequel est disposé en agencement de détecteurs (7) et un dispositif d'évaluation (8) qui est en liaison avec l'agencement de détecteurs, l'agencement de filtres présentant un premier filtre (9) et un second filter (10), lesquels sont réalisés en tant que filtres passe-bande et présentent respectivement une bande passante, et le premier filtre d'entre ces filtres laissant passer une bande IR prédéfinie et le second filtre de ces filtres ne la laissant pas passer, et l'agencement de détecteurs présentant deux détecteurs (14, 15), chacun d'entre eux étant associé à un filtre, la bande passante (F1) d'un filtre étant disposée à l'intérieur de la bande passante (F2) de l'autre filtre et le dispositif d'évaluation pouvant former une différence entre les signaux des détecteurs, **caractérisé en ce que** les deux filtres (9, 10) sont formés par des éléments de filtre (11, 13 ; 12, 13) consécutifs, un élément de filtre (13) étant identique pour les deux filtres (9, 10) et définissant une longueur d'onde de coupure (L) commune, et le dispositif d'évaluation (8) pouvant normaliser la différence entre les signaux (S1, S2) sur le signal d'un détecteur (14).

2. Détecteur IR selon la revendication 1, **caractérisé en ce que** la bande passante (F1) du premier filtre (9) est plus grande que la bande passante (F2) du second filtre (10).

3. Détecteur IR selon la revendication 1 ou 2, **caractérisé en ce que** les deux filtres (9, 10) présentent la même longueur d'onde de départ (L).

4. Détecteur IR selon l'une des revendications 1 à 3, **caractérisé en ce que** le premier filtre (9) présente une bande passante (F1), laquelle est plus grande de l'ordre de 0,3 à 0,7 $\mu$m que la bande passante (F2) du second filtre (10).

5. Détecteur IR selon la revendication 4, **caractérisé en ce que** le premier filtre (9) présente une bande passante dans la plage de 3,6 à 4,5 $\mu$m et le second filtre (10) présente une bande passante (F2) dans la plage de 3,6 à 4,0 $\mu$m.

6. Détecteur IR selon e 'une de revendications 1 à 5, **caractérisé en ce qu'**il peut utiliser le rayonnement naturel à partir du milieu.

7. Détecteur IR selon l'une des revendications 1 à 6, **caractérisé en ce que** le dispositif d'évaluation (8) peut normaliser la différence sur le signal (S1) du premier détecteur (14).

8. Détecteur IR selon l'une des revendications 1 à 7, **caractérisé en ce que** les filtres (9, 10) présentent du $CaF_2$, du germanium ou du silicium

# Fig.1

# Fig.2

# Fig.3

# Fig.4

# Fig.5

# Fig.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5081998 A
- US 6369716 B1
- US 2003147080 A1
- US 5995008 A